# EUROPEAN PATENT APPLICATION

(11) **EP 2 025 334 A1**
(43) Date of publication of application: **18.02.2009**
(21) Application number: 07113152.8
(22) Date of filing: 25.07.2007
(51) Int. Cl.: A61K 9/16

(54) **Sustained release drug delivery system for repeated administration**

(71) Applicant: OctoPlus Sciences B.V., 2333 CL Leiden (NL)
(72) Inventor: Verrijk, Rudolf, 2202 HS Noordwijk (NL); De Leede, Leonardus Gerardus Josef, 2743 HM Waddinxveen (NL)
(74) Representative: van Loon, C.J.J.

(57) **Abstract**

Described is a delivery system for the sustained release of a drug, comprising a carrier and a drug, which provides for a pseudo zero order release upon multiple administration. Thus, the system is for use in a method of treatment comprising the repeated administration of the drug, wherein the release follows a sigmoidal pattern over an Administration Interval between a first and a second administration, and wherein the delivery system provides for a remaining load of the drug at the end of the Administration Interval. Preferably the remaining load is such as to provide a level of release which, in sum with the initial release of a next sigmoidal release administration, provides for a release level approximating plateau release.

## Description

The invention pertains to a delivery system for the sustained release of a drug, comprising a carrier and a drug, for use in a method of treatment comprising the repeated administration of the drug.

In providing sustained release of a drug, it is frequently sought to obtain zero order release. A theoretical perfect zero order release will follow a linear release pattern, without an initial peak and without a decline before the releasable amount of the drug in the system is exhausted. Developments in such systems generally focus on avoiding a burst release shortly after administration, and avoiding declined release near the end.

Another type of release pattern has a more or less s-shaped curve, which is denoted sigmoidal or sigmoid release. Sigmoidal release generally involves an initial lag time during which no drug, or very little drug is released, followed by a phase where the rate of drug release increases, followed by a phase where the rate of drug release decreases toward zero. A reference in this regard is OA 12348 which describes the sigmoidal release of a specific drug, viz. eletriptan.

Another reference in the area of sigmoidal release is US 4,749,576. This relates to the use of swellable hydrogels as a matrix for a drug, and describes how to obtain sigmoidal release curves from this matrix.

A reference regarding selecting linear or sigmoidal release profiles is US 2003/0203039 which pertains to the preparation of microparticles as a carrier for controlled drug release.

The invention puts to use the sigmoidal release of a drug, in such a way as to provide advantages upon the repeated administration of the drug.

To this end, the invention provides for a system as described in the opening paragraph, wherein the release follows a sigmoidal pattern over an Administration Interval between a first and a second administration, and wherein the delivery system provides for a remaining load of the drug at the end of the Administration Interval.

The end of the Administration Interval is defined with reference to the point in time of the second administration. Thus, the invention also provides for a delivery system for the sustained release of a drug, comprising a carrier and a drug, for use in a method of treatment comprising the repeated administration of the drug, wherein the release follows a sigmoidal pattern over an Administration Interval between a first and a second administration, and wherein the delivery system provides for a remaining load of the drug at the point in time of the second administration.

The concept of a first administration referred to, is not necessarily the first administration of a treatment regimen. It is preferred if the invention applies to the first Administration Interval of a treatment regiment, and more preferred if it applies to substantially all of the subsequent Administration Intervals of such regimen. It is possible, however, that a treatment regimen includes an initial period of one or more Administration Intervals that do not have a sigmoidal release pattern and/or remaining load according to the invention.

The sigmoidal release pattern during the Administration Interval preferably is such as to have an Early Release per time-unit below Theoretical Release per time-unit. The time-unit can be hours, days or weeks and will be dependent on the application, drug and route of administration. By another preference, the sigmoidal release pattern is such as to have an Early Release followed by a period of approximately constant release providing a Plateau Release per time-unit, wherein the Early Release per time-unit is below the Plateau Release per time-unit.

"Theoretical Release per time-unit" indicates the amount of drug incorporated into a single dosage, divided by the number of time-units of the Administration Interval.

"Plateau Release per time-unit" indicates the release per time-unit associated with a period of more or less constant highest release rate exhibited during the part of the Administration Interval between the period of Early Release, and before a period of declined release during a terminal portion of the Administration Interval.

The "Early Release" indicates the initial period after administration, in which (as typical for sigmoidal release) a relatively low release takes place. The duration of the period of Early Release, and the amount of drug released, will differ per drug and per route of administration. As to duration, the Early Release period will generally be up to one third of the Administration Interval, preferably of from 5% to 25% of the duration of the Administration Interval, and more preferably of from 10% to 15% thereof. The amount of drug per time-unit will generally be up to 25% of the total amount, preferably up to 15% of the early release period.

The "Average Release per time-unit" indicates the total amount of drug released during the Administration Interval, divided by the number of time-units of the Administration Interval. If all of the drug administered were released within the Administration Interval, the Average Release per time-unit will be equal to the Theoretical Release per time-unit. If not all of the drug administered is released within the Administration Interval, the Average Release per time-unit will be lower than the Theoretical Release per time-unit. In the latter case, which is in accordance with the invention, the quantity of drug not released within the Administration Interval, is referred to as a "Remaining Load."

The concept of "Sustained Release" as used in the present invention refers to administration systems which do not provide immediate release of drug, but which are capable of releasing a controlled amount of a drug over a period of time, (the "Administration Interval"). The Administration Interval is dependent on the route of administration. For oral administration, administration is preferred as once or twice a day. For parenteral administration preferably more than one week, e.g. 2-4 weeks, and most preferably it is 2 weeks, 4 weeks, or 1 month. For dermal and transdermal administration, administration intervals of days to a week are preferred.

The invention is not limited to any specific system for the Sustained Release of drug, as long as it results in the aforementioned release characteristics. E.g., the system can be in accordance with US 4,749,576 or US 2003/0203039, mentioned above.

Preferably, the system is based on hydrogel or on micro particle technology. In the latter case, it is preferred to use microparticles technology as described in EP941068 or as in WO 2006/085747. In the former case, it is preferred to use hydrogel technology as described in or EP 910412. These disclosures are referred to rather than repeated here, and the entire content thereof is incorporated herein by reference.

It is within the ambit of the person skilled in the art to manufacture the desired sustained release preparations and obtain sigmoidal release. Release can be determined in various ways known in the art, and for generally used drugs, appropriate in vitro release determinations exist.

As mentioned above, the system provides for a Remaining Load. This refers to systems still capable of releasing drug at the end of the Administration Interval. The Remaining Load preferably comprises 10-50 wt.% of the initial drug load in the system, and more preferably 15-35 wt.%.

The Remaining Load can be provided for by selecting the duration of the Administration Interval such as to be shorter than the period of time during which all of the drug is released. Based on a given Administration Interval, the Remaining Load is realized by structuring the carrier loaded with drug in such a way as to avoid that all of the drug releasable therefrom will in fact be released over the period of time of the Administration Interval. The exact way in which this can be done, and which processing conditions to choose (e.g. a higher or lower drug polymer ratio, selecting of a solvent for the drug during polymer encapsulation with a higher or lower drug solubility or a faster or slower evaporation of solvent of the polymer during the systems production process so as to influence the amount of drug near the surface or near the core of the carrier), will differ per drug and per delivery system. The knowledge, in accordance with the invention, to combine a sigmoidal release pattern with a Remaining Load, will be sufficient for the person skilled in the art to set the right conditions without undue experimentation.

The concept of a sigmoidal release during the Administration Interval, in combination with a Remaining Load as described, serves to provide a more constant release over a multitude of repeated administrations. The repeated administrations will be at least two, but the benefits of the present invention will become more apparent upon a higher number of repeated administrations. There will generally be no limit to the number of repeated administrations other than as determined by the safety and efficacy, and ultimately the administration regimen as prescribed, of the drug in question. In the system of the invention, at the point in time of a second (or further) administration, the Remaining Load of the first (or previous) administration will serve to complement the Early Release of said second or further administration, so as to reach a desired level of combined release. If desired, the level of combined release can be substantially below or above Plateau Release. The benefit of the present invention will be mostly enjoyed if the level of combined release is not too much below or above the Plateau release. Thus, the sum of the Remaining Release of a first (or previous) administration and the Early Release of a second (or further) administration is 65-135% of the Plateau Release. Most preferably, said sum approximates Plateau Release.

In addition to providing a more constant release pattern over multiple administrations, the invention allows a benefit in obtaining more constant levels (as determined in serum or tissue, as applicable) of the drug administered (or a relevant metabolite, as applicable). Particularly, the difference between peak and trough levels for the tissue or blood compartment drug concentration can be substantially decreased as a result of the present invention. In certain areas of treatment, e.g. in treating cancer with drugs that exhibit uncomfortable or even inhibitory side effects if high peak levels are reached. Similarly, therapies exist, e.g. in treating viral infections, in which it is desirable to avoid steep drug level nadirs, and/or to avoid the frequency of such nadirs. It is believed that reducing the occurrence of drug nadirs may improve the likelihood of what is called a sustained viral response (i.e. in which patients have a better chance of the viral infection not recurring).

The invention be used for the sustained release of any drug for which sustained release is suitable. In particular for drugs with a small therapeutic margin, where the ratio between the desired therapeutically needed serum or tissue concentration and the concentration where toxicity is observed. Also in treating infections where low serum or tissue drug concentrations would lead to the undesired formation of resistance of the micro-organism against the drug.

The invention will be further illustrated with reference to the following non-limiting Figures and Examples.

### Example 1

Theoretical curves, making use of the principles (sigmoidal shape and significant final load (surplus) at time=14), compared to the situation without the principle.

Three formulations are presented as examples
A as pseudo-zero order reference with approximately 5% surplus loading at time = 14.
B as a formulation with the "sigmoidal" principle and a surplus loading at time = 14 of approximately 20%.
C as a formulation with the "sigmoidal" principle and a surplus loading at time = 14 of approximately 40%.

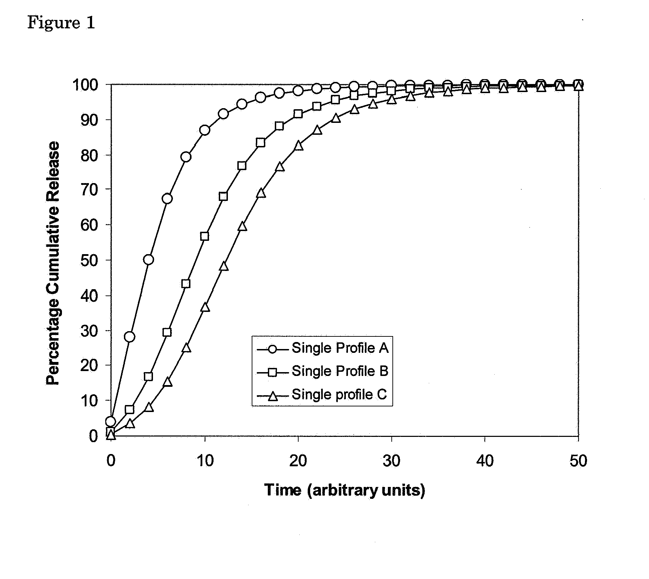

Figure 1 Theoretical drug release curves, illustrating the principles of a slowly increasing release rate (B and C) vs a pseudo-zero order release rate (A). Situation after single administration

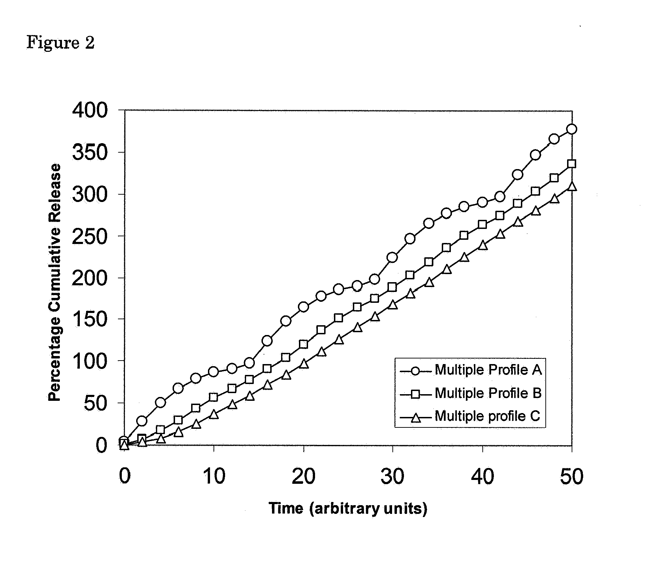

Figure 2 Theoretical drug release curves, illustrating the principles of a slowly increasing release rate (B and C) vs a pseudo-zero order constant release rate (A). Situation after multiple administrations at t = 0, t = 14 and at subsequent multiples of time = 14. Note the non-linearity of Profile A vs the linear profiles of B and C.

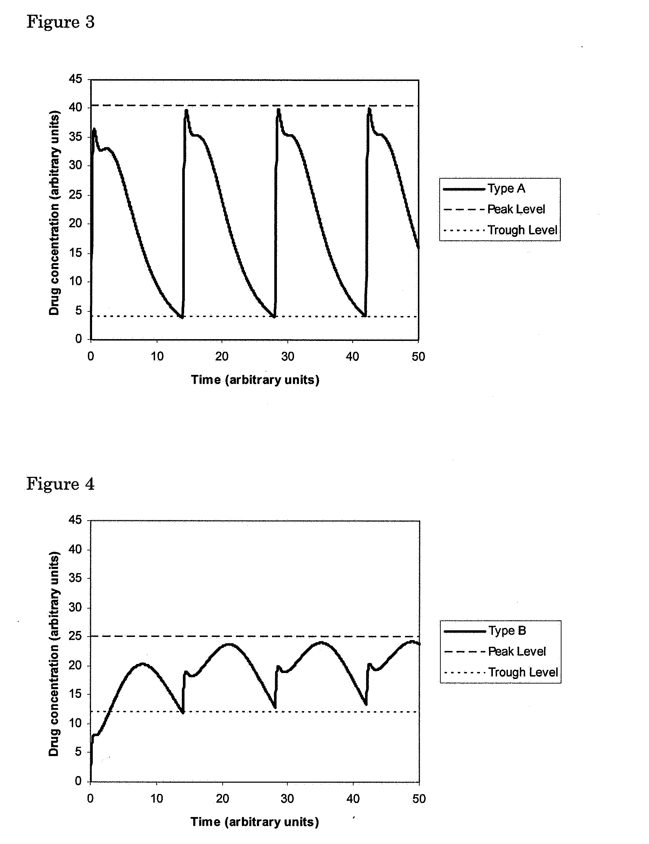

Figure 3. Calculated prediction of tissue or blood compartment drug concentration during 50 arbitrary time units, after administering formulation A at t = 0, t = 14 and at subsequent multiples of time = 14. The peak/trough ratio of the drug concentration is 9.7. The drug is a high clearance compound with an absorption half life of t = 0.2 , an elimination half life of t= 0.1 and an apparent volume of distribution of 95 litre.
Figure 4. Calculated prediction of tissue or blood compartment drug concentration during 50 arbitrary time units, after administering formulation B at t = 0, t = 14 and at subsequent multiples of time = 14. The peak/trough ratio of the drug concentration is 1.8. The drug is a high clearance compound with an absorption half life of t = 0.2 , an elimination half life of t= 0.1 and an apparent volume of distribution of 95 litre.

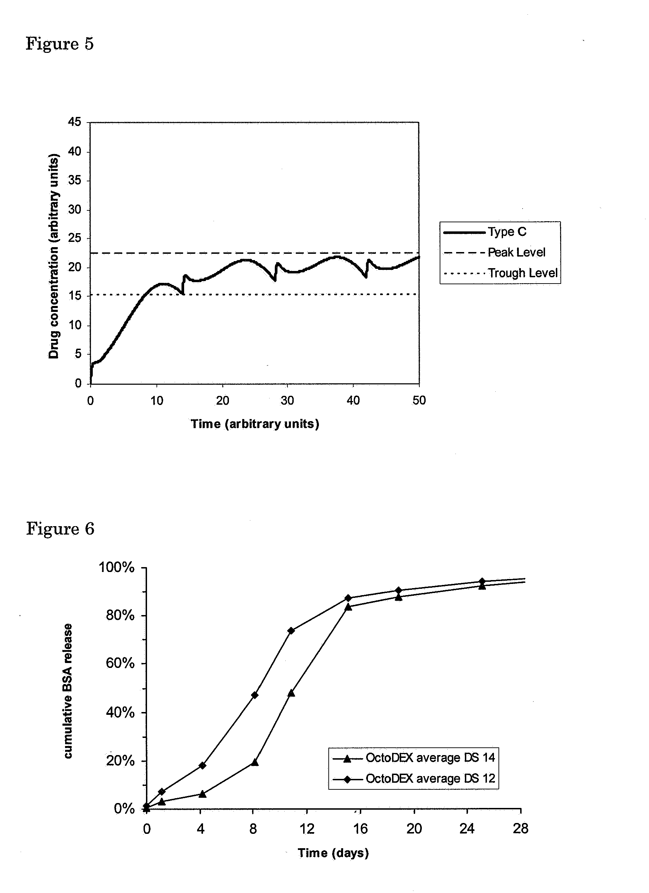

Figure 5. Calculated prediction of tissue or blood compartment drug concentration during 50 arbitrary time units, after administering formulation C at t = 0, t = 14 and at subsequent multiples of time = 14. The peak/trough ratio of the drug concentration is 1.2. The drug is a high clearance compound with an absorption half life of t = 0.2 , an elimination half life of t= 0.1 and an apparent volume of distribution of 95 litre.

### Example 2

Figure 6. Sigmoidal-shaped release profiles from BSA-loaded OctoDEX microspheres

### Preparation of BSA(Bovine Serum Albumin)-loaded microspheres

Protein-loaded dex-HEMA microsphere batches were prepared by a water-in-water emulsion technique. All used solutions were based on 25 mM phosphate buffer pH 7.0..

For the preparation of BSA-loaded microspheres, a solution containing dex-HEMA (61 mg; DS12) and BSA (6.1 mg - 10% (w/w) in relation to dex-HEMA), was added to a PEG 10,000 solution (40% w/w). The ratios (w/w) between the added PEG solution and the dex-HEMA-protein solution (together 5.0 g) were estimated from phase diagrams of PEG 10,000 and dex-HEMA, aiming for emulsions with a PEG/dex-HEMA volume ratio of 40 and a 50% (w/w) water content of the dex-HEMA-protein phase. The resulting two-phase system was mixed vigorously with a vortex for 1 min. The resulting emulsion was allowed to stabilise for at least 15 minutes. Polymerisation of the dex-HEMA was initiated by subsequent addition to the emulsion of solutions of TEMED (100 µl, 20% v/v, 1.6 M HCl) and Potassium persulfate (180 µl, 50 mg/ml). All solutions contained 25 mM phosphate buffer pH 7.0. The reaction vessels were left at room temperature (without stirring) for at least 1 hour. The protein-loaded dex-HEMA microspheres were collected and purified by three cycles of centrifugation (4500 rpm; first cycle 15 min, thereafter 5 min) and washing with physiological buffer. The encapsulation efficiency of BSA was calculated from the amount of BSA measured in the washing fractions.

In vitro release was performed in Phosphate Buffer Saline at 37°C. Samples were analysed for monomeric BSA by size-exclusion chromatography with UV detection.

## Claims

1. A delivery system for the sustained release of a drug, comprising a carrier and a drug, for use in a method of treatment comprising the repeated administration of the drug, wherein the release follows a sigmoidal pattern over an Administration Interval between a first and a second administration, and wherein the delivery system provides for a remaining load of the drug at the end of the Administration Interval.

2. A delivery system for the sustained release of a drug, comprising a carrier and a drug, for use in a method of treatment comprising the repeated administration of the drug, wherein the release follows a sigmoidal pattern over an Administration Interval between a first and a second administration, and wherein the delivery system provides for a remaining load of the drug at the point in time of the second administration.

3. A delivery system according to claim 1 or 2, wherein the remaining load is 10-50% of the initial drug load in the system.

4. A delivery system according to claim 3, wherein the remaining load is 15-35% of the initial drug load in the system.

5. A delivery system according to any one of the preceding claims, wherein the sigmoidal release pattern comprises an Early Release below Plateau Release and a Remaining Load providing a Remaining Releaese, wherein the sum of the Remaining Release of a first administration and the Early Release of a second administration is 65-135% of the Plateau Release.

6. A delivery system according to claim 5, wherein said sum approximates Plateau Release.

7. A delivery system according to any one of the preceding claims, wherein the carrier is selected from the group consisting of hydro gels and microparticles.

8. A delivery system according to claim 7, wherein the hydrogel is a biodegradable polymer based on dextran or derivatised dextran, or based on Polyetherester Copolymers

9. A delivery system according to claim 8, wherein the microparticles are 2-150 um microspheres made of biodegradable polymers based on dextran or derivatised dextran, or based on Polyetherester Copolymers
